(19) Europäisches Patentamt
European Patent Office
Office européen des brevets

(11) **EP 1 551 288 B1**

(12) **FASCICULE DE BREVET EUROPEEN**

(45) Date de publication et mention
de la délivrance du brevet:
**07.12.2011 Bulletin 2011/49**

(21) Numéro de dépôt: **03767930.5**

(22) Date de dépôt: **14.10.2003**

(51) Int Cl.:
***A61B 5/0476*** (2006.01) ***G06F 17/00*** (2006.01)

(86) Numéro de dépôt international:
**PCT/FR2003/050090**

(87) Numéro de publication internationale:
**WO 2004/035130 (29.04.2004 Gazette 2004/18)**

(54) **PROCEDE D ANALYSE ET DISPOSITIF DE SUIVI MEDICAL OU COGNITIF EN TEMPS REEL A PARTIR DE L ANALYSE DE L ACTIVITE ELECTROMAGNETIQUE CEREBRALE D UN INDIVIDU, APPLICATION DE CE PROCEDE POUR CARACTERISER ET DIFFERENCIER DES ETATS PHYSIOLOGIQUES OU PATHOLOGIQUES**

VERFAHREN ZUR ANALYSE UND VORRICHTUNG ZUR MEDIZINISCHEN ODER KOGNITIVEN ECHTZEIT-ÜBERWACHUNG DER ELEKTROMAGNETISCHEN ZEREBRALEN AKTIVITÄT EINES INDIVIDUUMS, VERWENDUNG DIESES VERFAHREN ZUR CHARAKTERISIERUNG UND BESTIMMUNG DER PHYSIOLOGISCHEN ODER PATHOLOGISCHEN ZUSTÄNDE

ANALYSIS METHOD AND REAL TIME MEDICAL OR COGNITIVE MONITORING DEVICE BASED ON THE ANALYSIS OF A SUBJECT'S CEREBRAL ELECTROMAGNETIC ACTIVITY, USE OF SAID METHOD FOR CHARACTERIZING AND DIFFERENTIATING PHYSIOLOGICAL OR PATHOLOGICAL STATES

(84) Etats contractants désignés:
**AT BE BG CH CY CZ DE DK EE ES FI FR GB GR HU IE IT LI LU MC NL PT RO SE SI SK TR**

(30) Priorité: **18.10.2002 FR 0213007**

(43) Date de publication de la demande:
**13.07.2005 Bulletin 2005/28**

(73) Titulaire: **CENTRE NATIONAL DE LA RECHERCHE SCIENTIFIQUE**
**75794 Paris Cedex 16 (FR)**

(72) Inventeurs:
• **MARTINERIE, Jacques**
**F-91120 PALAISEAU (FR)**
• **LE VAN QUYEN, Michel**
**F-75015 PARIS (FR)**
• **LACHAUX, Jean-Philippe**
**F-92340 Bourg-la-Reine (FR)**
• **RENAULT, Bernard**
**F-75012 PARIS (FR)**

(74) Mandataire: **Jacquard, Philippe Jean-Luc**
**Cabinet ORES**
**36, rue de St Pétersbourg**
**75008 Paris (FR)**

(56) Documents cités:
WO-A-00/10455    WO-A-01/37724
US-A- 4 201 224    US-A- 4 846 190
US-A1- 2002 042 563    US-B1- 6 304 775

• LACHAUX ET AL.: "Measuring Synchrony in Brain Signals" HUMAN BRAIN MAPPING, vol. 8, 1999, pages 194-208, XP002247405
• QUIROGA R Q ET AL: "Event synchronization: a simple and fast method to measure synchronicity and time delay patterns" PHYSICAL REVIEW E (STATISTICAL, NONLINEAR, AND SOFT MATTER PHYSICS), OCT. 2002, APS THROUGH AIP, USA, vol. 66, no. 4, octobre 2002 (2002-10), pages 41904-1-9, XP002247968 ISSN: 1063-651X
• LE VAN QUYEN M ET AL: "Comparison of Hilbert transform and wavelet methods for the analysis of neuronal synchrony" JOURNAL OF NEUROSCIENCE METHODS, 30 OCT. 2001, ELSEVIER, NETHERLANDS, vol. 111, no. 2, 30 octobre 2001 (2001-10-30), pages 83-98, XP002248012 ISSN: 0165-0270

**Description**

Domaine technique

La présente invention concerne un procédé et un dispositif de suivi médical ou cognitif en temps réel à partir de l'analyse de l'activité électromagnétique cérébrale d'un individu, avec des applications de ce procédé pour la différenciation et la caractérisation d'états physiologiques et pathologiques, notamment pour le traitement en temps réel de l'anticipation des crises d'épilepsie.

Etat de la technique antérieure

Rôle des interactions cérébrales chez l'homme : la cartographie dynamique

Tout acte cérébral émerge d'une coopération entre plusieurs réseaux neuronaux spatialement distribués. A l'heure actuelle, et malgré leurs progrès récents, les principales techniques d'imagerie cérébrale (EEG (électroencéphalographie), MEG (magnétoencéphalographie), IRMF (imagerie par résonance magnétique fonctionnelle) et TEP (tomographie par émission de positions)) ne fournissent qu'une cartographie des activations cérébrales, sans rendre directement compte des interactions entre ces activations. La caractérisation de ces réseaux fonctionnels nécessite à la fois :

- l'identification des régions cérébrales impliquées ;
- la compréhension des mécanismes d'interaction entre ceux-ci ; et
- la quantification précise de ces interactions.

L'observation du fonctionnement de ces réseaux n'est pas possible à partir de la seule cartographie des activités cérébrales. En effet, comment savoir, parmi toutes les zones actives simultanément, celles qui participent à un même réseau ? La simple observation que deux zones sont actives en même temps ne suffit pas pour conclure qu'elles sont engagées dans le même processus pathologique ou cognitif. Pour élucider ces mécanismes d'échange, il est nécessaire d'avoir des hypothèses explicites sur la nature de ces liens et de disposer de moyens techniques pour les observer.

Toutes les approches de l'art connu s'appuient sur l'idée que l'existence d'un couplage entre deux zones doit se traduire par une corrélation entre leurs activités. Or, l'activité d'un groupe de neurones, par exemple une colonne corticale, peut se caractériser par deux types de mesure :

- un codage temporel avec le taux de décharges neuronales par seconde ; ou
- un codage par la synchronisation des activités oscillatoires des zones cérébrales impliquées dans un même réseau.

L'invention a pour objet de proposer un procédé de cartographie dynamique du cerveau à partir d'une telle mesure de synchronie de phase, en partant de l'hypothèse selon laquelle les liens dynamiques entre les groupes neuronaux se manifestent par la synchronisation d'activité oscillatoire dans certaines bandes de fréquence entre 0 et 2000 Hz.

Le document US 4,846,190 décrit un système d'analyse et affichage de données électroencéphalographiques. Ce système permet, entre autre, le calcul de la cohérence entre les signaux électroencéphalographiques engendrés par des régions « homologues » du cerveau d'un patient, et la génération d'un signal d'alerte lorsqu'une anomalie est détectée.

Exemple d'application à la pathologie : anticipation des crises d'épilepsie

Dans la suite de la description, le procédé de l'invention est appliqué, à titre d'exemple, et pour la clarté de l'exposé, au suivi médical d'un patient pour l'anticipation en temps réel de crises d'épilepsie. Il est, bien entendu, possible d'appliquer le procédé de l'invention dans d'autres domaines et de caractériser, puis reconnaître des états physiologiques ou pathologiques en changeant la base de données.

L'épilepsie, une des affections neurologiques les plus fréquentes de l'enfant et de l'adulte (1 % de la population), est la conséquence d'un désordre neuronal qui s'exprime par des décharges paroxystiques, récurrentes, du cortex cérébral. La traduction clinique en est la survenue soudaine des symptômes d'une crise. Cette émergence soudaine est difficile à interpréter comme une réponse à un facteur externe déclenchant, qui est absent dans la plupart des situations, excepté les rares épilepsies réflexes. La transition entre l'état dit « intercritique » et l'état critique (la crise) est une des phénoménologies primordiales de l'épilepsie, et cette intermittence apparaît comme le produit imprévisible d'une auto-organisation interne au cerveau.

Aucune méthode traditionnelle (analyse linéaire) n'avait permis jusqu'à présent une anticipation significative de cet état de crise.

Deux publications récentes, référencées [1] et [2] en fin de description, décrivent une procédure qui permet, dans certaines conditions, d'anticiper les crises de plusieurs minutes à l'aide de nouvelles stratégies issues de la théorie des systèmes dynamiques. Les méthodes de la dynamique non linéaire dérivent des mathématiques connues sous le nom générique de « Théorie du Chaos ». Elles permettent de révéler comment, derrière un signal électroencéphalographique en apparence aléatoire, peuvent se cacher des lois ou des déterminismes précis. La possibilité d'anticiper la survenue de crises ouvre des perspectives très vastes.

L'anticipation des crises s'adresse aux nombreux patients présentant une épilepsie pharmacorésistante, ce qui représente environ 25 % des patients épileptiques. La survenue intermittente et inattendue des crises est un facteur reconnu de mortalité (par accidents de voiture, ou mort subite) et de morbidité (notamment traumatismes crâniens et faciaux). De plus, le caractère imprévisible des crises est considéré par les patients comme un des plus importants facteurs de mauvaise qualité de vie. Les limitations liées au risque de crises sont d'ordre social (isolement lié à la peur de faire une crise en public), professionnel (métiers à risques interdits aux épileptiques) et personnel (conduite automobile interdite). La possibilité d'anticiper la survenue des crises serait un moyen d'aider les épileptiques à vivre mieux avec leurs crises. En plus de cette possibilité d'alerter le patient de l'imminence d'une crise, les stratégies d'anticipation devraient également aider à la réalisation d'examens complémentaires dans le cadre d'un bilan pré-chirurgical de leur épilepsie. D'autre part, de telles stratégies devraient ouvrir la voie à des interventions thérapeutiques avant même que la crise n'ait le temps d'émerger.

Le document référencé [1], cité ci-dessus, repose sur la quantification de la différence de similarité entre une dynamique de référence « normale » et la dynamique émergente épileptique. Cet indice de similarité est calculé indépendamment pour chacune des voies enregistrées. Les composantes spatiales et temporelles de la dynamique cérébrale peuvent être obtenues en alignant les évolutions de la déviation statistique des indices de chacune des voies. Néanmoins, cette méthode ne prend qu'indirectement en compte l'aspect spatial des modifications de la dynamique de l'électroencéphalographie (intracrânien et de surface) qui nous permettent de prédire l'imminence d'une crise. De fortes évidences suggèrent toutefois que cette période reflète la transition d'un état désordonné vers un état plus ordonné (ou moins complexe) qui sont fortement susceptible de correspondre à des changements de synchronisation de plusieurs ensembles neuronaux distribués dans différentes structures cérébrales. Pour augmenter l'efficacité de l'anticipation, l'idéal serait d'avoir des informations supplémentaires concernant les comportements spatio-temporels de la dynamique épileptogène, telles que des subtiles variations de l'activité ou les interdépendances entre régions cérébrales distantes. De nombreuses observations suggèrent en effet que la détermination d'une zone de dysfonctionnements épileptiques unique de l'origine d'une crise est souvent très délicate. En particulier, de récents travaux ont largement argumenté l'importance de concevoir les épilepsies partielles comme une manifestation d'une structure en réseau. En effet, il est vraisemblable que la propagation de la décharge facilite un certain nombre de connexions entre de multiples ensembles neuronaux, modifiant ainsi d'une façon plus performante des connexions neuronales locales et à distance. Ainsi, l'organisation des dysfonctionnements épileptiques ne peut plus être envisagée de manière statique ou comme un dysfonctionnement local (foyer épileptique circonscrit) mais répondre à un modèle dynamique patio-temporel complexe, impliquant spatialement des réseaux neuronaux reliés par des connexions anormalement facilitées et mettant en jeu au cours du temps certains comportements synchronisés.

Pour étudier l'organisation spatiale du réseau épileptogène et caractériser les interactions entre le réseau épileptogène et le reste du cerveau, les techniques linéaires (cross-corrélations dans le domaine temporel ou de cohérences dans le domaine fréquentiel) ont souvent été employées dans le passé. Ces méthodes sont souvent, dans le cas de l'analyse entre signaux macroscopiques, limitées par leurs hypothèses concernant la stationnarité des signaux ainsi que la nature linéaire des interactions. Ceci est d'autant plus problématique, qu'en période épileptique, on voit se renforcer nettement les comportements non linéaires.

La publication [4] décrit deux méthodes d'analyse de la synchronie de signaux neuronaux, et notamment la méthode (S)PLS mise en oeuvre par un procédé selon un mode de réalisation de l'invention.

L'invention a pour objet de dépasser les limitations de l'art antérieur.

## Exposé de l'invention

L'invention concerne un procédé d'analyse des synchronisations de l'électroencéphalographie d'un .ndividu en utilisant un ensemble de capteurs à partir le l'analyse électromagnétique cérébrale de celui-ci, caractérisé en ce qu'il comprend les étapes suivantes :

- une étape de constitution d'une base de données comprenant :

- • une phase d'acquisition et de numérisation de signaux électrophysiologiques issus de ces capteurs,
- • une phase de calcul d'une corrélation entre des variations temporelles de phases instantanée entre toutes les paires de capteurs enregistrés dans un protocole de montage, dans des bandes de fréquences comprises entre 0 et 2000 Hz, pour constituer cette base de données de classes caractérisant chacune un état de référence;

- - une étape de validation statistique d'une période analysée en temps réel basée sur une méthode de discrimination non paramétrique multidimensionelle, qui permet d'affecter cette période à une classe de la base de données,
- - une étape de détection d'une période spécifique.

Avantageusement, ledit procédé comprend une analyse associée à au moins l'un des types de signaux électrophysiologiques suivants : électrocardiogrammes, électroocculogrammes, électrodermogrammes, signaux de respiration.

Avantageusement, lors de l'étape de validation statistique, on utilise une méthode PLS, qui estime la différence de phase entre les oscillations des signaux de deux électrodes. Le niveau statistique de la synchronisation PLS entre deux signaux est évalué à l'aide de la variance circulaire de la différence de phase entre les signaux ou à l'aide de l'entropie normalisée de Shannon de la différence de phase entre les signaux.

Le procédé de l'invention peut être utilisé pour caractériser et différencier des états physiologiques ou pathologiques, par exemple pour l'anticipation de crises d'épilepsie.

Le procédé de l'invention peut s'appliquer à d'autres domaines d'application, tels que :

- - au sommeil : différenciation entre les différents stades de sommeil ;
- - à l'anesthésie : caractérisation des stades d'endormissement sous anesthésie avec un contrôle automatique de la régulation de la substance injectée ;
- - à la dépression : avec le suivi électrophysiologique d'un malade dépressif et la caractérisation de ses traits ou états et, en conséquence, l'ajustement de son traitement ;
- - à la schizophrénie : avec le suivi électrophysiologique d'un malade et la quantification de ses traits ou états à des fins d'aide au diagnostic et à la thérapeutique.
- - à l'aide du diagnostic pour les maladies neurologiques telles que : Parkinson, Alzheimer.
- - à la caractérisation d'états cognitifs (niveaux de vigilance et d'attention, de perception et de reconnaissance consciente et non consciente de stimulations visuelles, auditives, somesthésiques et également émotionnelles (peur, joie, etc.).

L'invention concerne également un dispositif de suivi médical ou cognitif en temps réel à partir de l'analyse électromagnétique cérébrale d'un individu, caractérisé en ce qu'il comprend :

- - des moyens d'acquisition et de numérisation de signaux électrophysiologiques issus de capteurs ;
- - des moyens de calcul, par une methode de discrimination non paramétrique moltidimensionnelle, entre toutes les paires de capteurs enregistrés dans un procédé de montage, dans des bandes de fréquences comprises entre 0 et 2000 Hz, pour constituer une base de données de classes caractérisant chacune un état de référence ;
- - des moyens de validation statistique, par une méthode de discrimination non paramétrique multimensionelle, d'une période analysée en temps réel qui permet d'affecter cette période à une classe de la base de données;
- - des moyens de détection d'une période cognitive ou d'une période pathologiques spécifique ;
- - des moyens d'émission éventuelle d'un signal alerte.

Avantageusement le dispositif de l'invention est un dispositif autonome, léger et transportable par le patient. Pour permettre aux sujets une autonomie complète, le dispositif de l'invention peut être miniaturisé pour pouvoir être implanté en sous-cutané, comme un stimulateur.

Brève description des dessins

- - La figure 1 illustre les différentes étapes du procédé de l'invention, à partir de l'analyse d'électroencéphalogrammes (EEGs).
- - La figure 2 illustre plus précisément les étapes du procédé de l'invention.
- - La figure 3 illustre le dispositif de l'invention.
- - La figure 4 représente des chronogrammes explicatifs du traitement des électroencéphalogrammes avec le procédé de l'invention.

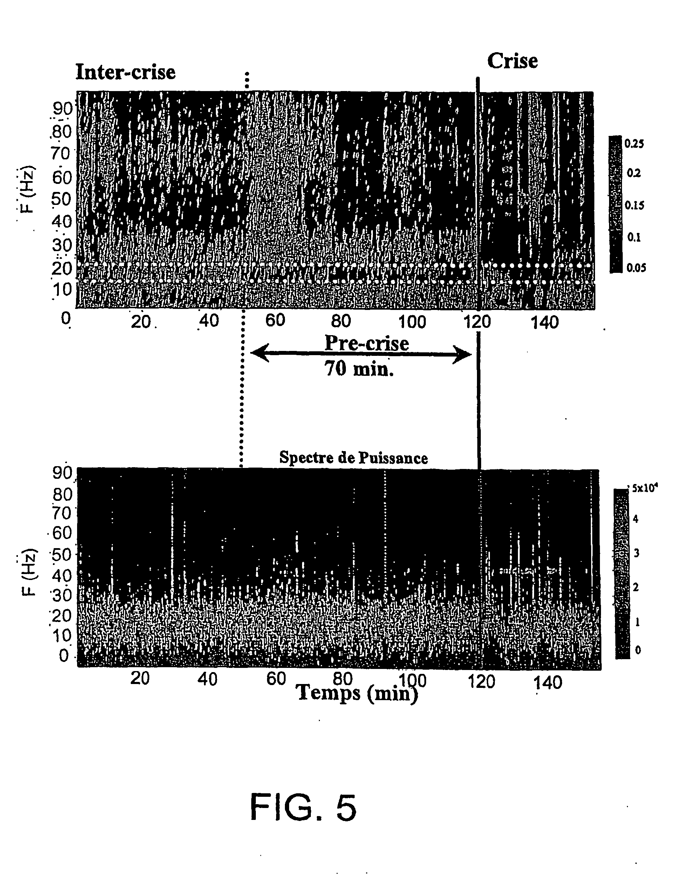

- La figure 5 illustre un exemple de mise en oeuvre du procédé de traitement en temps réel de l'épilepsie selon l'invention.

Exposé détaillé de modes de réalisation particuliers

L'activité neuroélectrique dans une bande de fréquence restreinte se caractérise par son énergie et sa phase, si bien que la mise en évidence d'une relation entre deux groupes neuronaux dans une certaine bande de fréquence passe par la démonstration d'une corrélation significative entre les variations de leurs énergies ou de leurs phases. La méthode la plus couramment employée utilise simultanément l'énergie et la phase. Elle consiste à calculer la cohérence entre les signaux selon l'indice de « Magnitude Squared Coherence » (MSC).

Cet indice MSC est une mesure globale dans laquelle il est difficile de séparer l'influence de la phase de celle de l'énergie. Or, une corrélation entre les variations des phases des deux signaux peut s'avérer suffisante pour démontrer un couplage entre deux groupes neuronaux (document référencé [3]).

Le procédé de l'invention permet une mesure de synchronie, en utilisant uniquement la phase : la méthode des « Phase Locking Statistics » (PLS). Pour une latence donnée, cette méthode estime la différence de phase entre les oscillations des signaux de deux électrodes. Si cette différence de phase reste relativement constante au cours de la période analysée, on obtient alors un indice PLS élevé, signe d'une synchronie significative entre les deux électrodes.

Cette méthode PLS est suffisamment précise pour détecter des périodes de synchronie et se révèle donc adaptée pour décrire une succession de synchronies transitoires comme celles supposées intervenir dans le traitement cognitif ou bien pour caractériser des synchronies plus soutenues comme celles supposées caractériser des états pathologiques.

Cette méthode permet de mesurer le degré de synchronisation entre les activités des diverses régions cérébrales. La synchronie entre deux groupes neuronaux se définit dans une certaine bande de fréquence comme une corrélation significative entre les variations temporelles de leur phase : il s'agit alors d'un accrochage de phase ou « phase-locking ». Néanmoins, compte tenu des effets de volume (l'activité d'une seule population neuronale peut ainsi être captée par deux électrodes relativement éloignées) et du bruit de fond neuronal, la détection d'une synchronie entre deux régions doit suivre une démarche statistique. La validité statistique des mesures est alors testée par la construction de données de remplacement bivariées.

L'invention peut ainsi utiliser une méthode d'estimation statistique basée sur l'emploi de données de remplacement (« surrogate data »), qui permet à la méthode PLS de s'appliquer, contrairement à la méthode MSC, à des signaux neuroélectriques non-stationnaires, comme c'est le cas de la plupart des signaux biologiques.

Le procédé de l'invention est donc un procédé de suivi médical ou cognitif en temps réel à partir de l'analyse de l'activité électromagnétique cérébrale d'un individu, associée à l'analyse éventuelle d'autres signaux électrophysiologiques (électrocardiogrammes, électroocculogrammes, électrodermogrammes, signaux de la respiration), pour notamment détecter des périodes cognitives, ou des périodes pathologiques, spécifiques, par exemple une crise d'épilepsie en préparation, et fournir dans tous les cas un signal d'alerte nécessaire pour permettre une prévention ou une intervention thérapeutique.

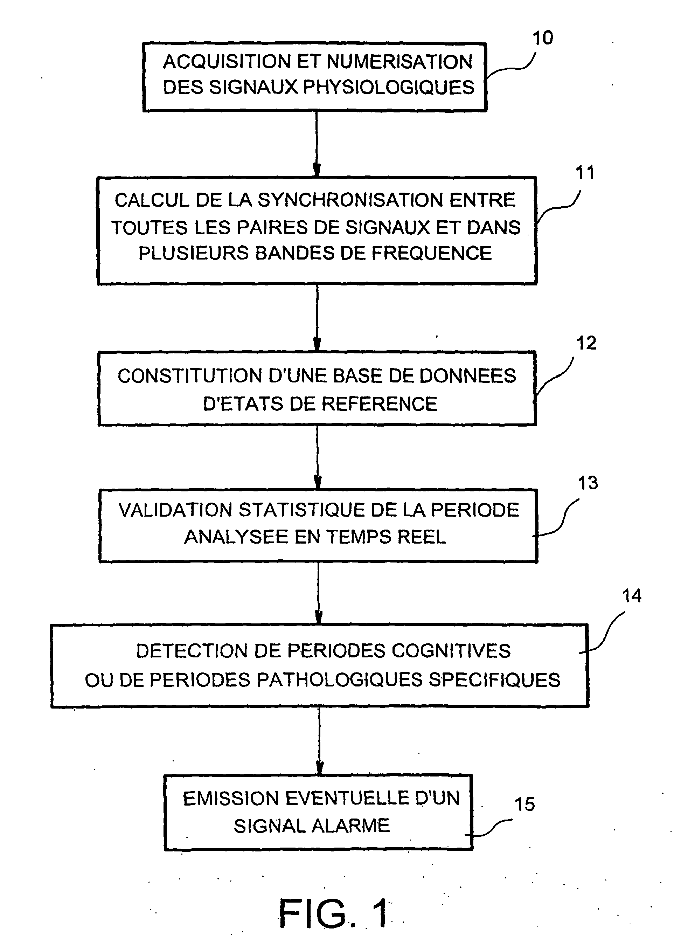

Comme illustré sur la figure 1, le procédé de l'invention comprend les étapes suivantes :

- une étape 10 d'acquisition et de numérisation des signaux électrophysiologiques : en général, un casque de 27 électrodes à 128 électrodes selon la problématique à résoudre, posé sur le scalp d'un individu, permet l'enregistrement de l'activité cérébrale de celui-ci avec une assez bonne résolution spatiale. Quelques signaux supplémentaires peuvent être acquis simultanément (signaux du mouvement des yeux, de l'activité cardiaque, etc.) ;
- une étape 11 de calcul de synchronisation entre toutes les paires des signaux et dans plusieurs bandes de fréquence pour constituer une base de données (étape 12) d'états de référence dépendant de la problématique mise en place (pathologiques, sommeil, veille, vigilance, etc.) ;
- une étape 13 de validation statistique de la période analysée en temps réel, qui permet de classer cette période à partir de la base de données. Cette validation repose sur une méthode de discrimination non paramétrique multidimensionnelle
- une étape 14 de détection de périodes cognitives ou de périodes pathologiques spécifiques ;
- une étape 15 éventuelle d'émission d'un signal alerte.

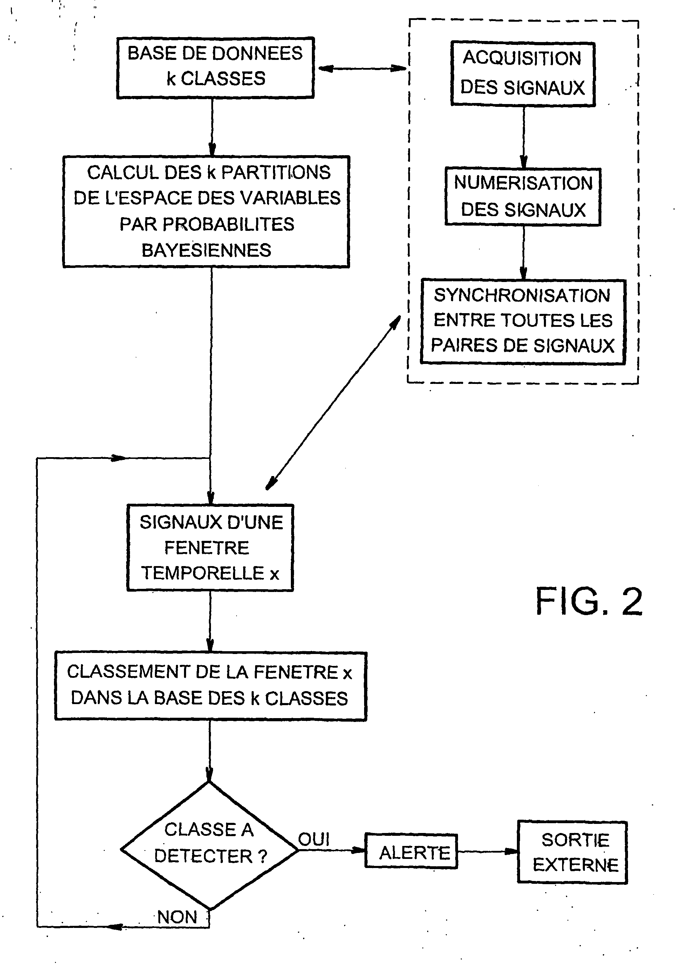

Plus précisément, comme illustré sur la figure 2, à partir d'une base de données de k classes, on peut avoir successivement :

- calcul de la partition de l'espace des variables par probabilités Bayésiennes $S_{ref}^{k}$ ;
- classement d'une fenêtre temporelle x, par exemple de 10 secondes, dans la base des k classes ;

- détection éventuelle d'une classe avec alerte.

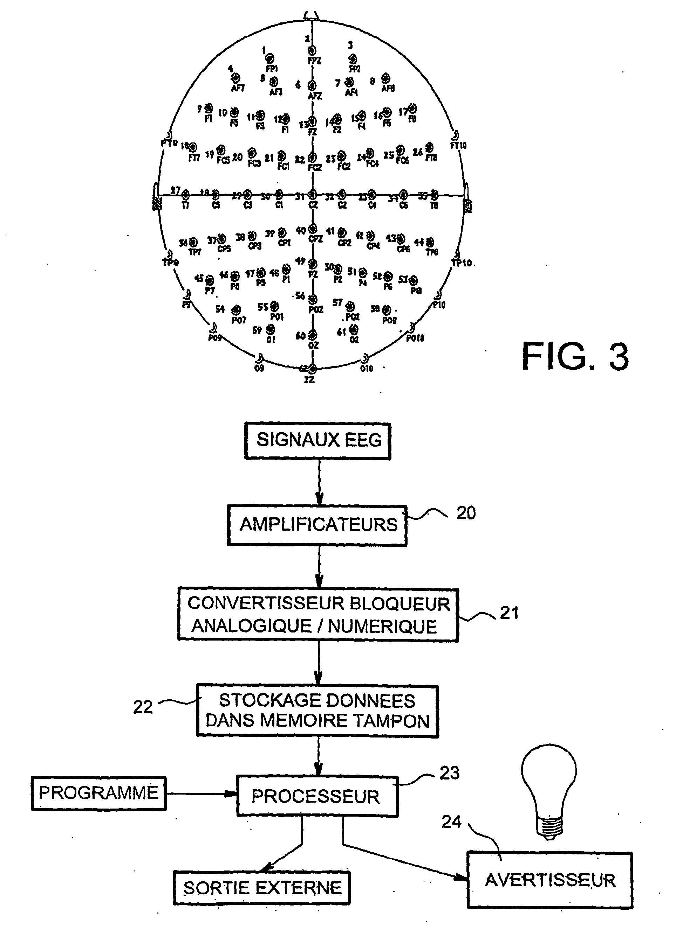

Comme illustré sur la figure 3, le dispositif autonome de suivi médical ou cognitif en temps réel à partir de l'analyse électromagnétique cérébrale d'un individu comprend des circuits d'acquisition (amplificateur 20, convertisseur analogique-numérique 21, tampon 22) des signaux de l'activité électrique du cerveau, un processeur 23 permettant l'acquisition et le traitement de ces signaux et un circuit d'alerte 24 pour le malade ou pour son environnement, par exemple un voyant.

Résultats attendus en épilepsie et implication aclinique

On a observé que certains couples d'électrodes dans la périphérie de la zone épileptogène présentent systématiquement, avant une crise, une modification significative de leur degré de synchronie, notamment par exemple dans la bande des fréquences alpha (8-12 Hz), bêta (15-30 Hz) et gamma (30-70 Hz). De manière intéressante, ces synchronisations ont récemment reçu une grande attention pour leur possible rôle dans les phénomènes d'intégration de large échelle pendant la cognition. Ces résultats suggèrent ainsi que les populations neuronales sous-jacentes à la zone épileptogène modifient, avant la crise, leurs relations avec une dynamique de plus grande échelle. Ces changements dans les synchronisations peuvent conduire à une « isolation dynamique » du foyer et pourraient fournir, de manière récurrente une population neuronale facilement recrutable par les processus épileptiques.

Les nouvelles techniques d'analyses des synchronisations de l'électroencéphalographie, utilisées dans le procédé de l'invention, permettent de quantifier très précisément l'activité cérébrale précritique. Cette possibilité d'anticiper la survenue de crises ouvre des perspectives médicales très vastes :

- en recherche fondamentale, par la caractérisation des modifications neurobiologiques qui surviennent pendant cette phase précritique ;
- en clinique, par la possibilité de prévenir le patient, et d'essayer de faire avorter la crise en préparation par une intervention thérapeutique.

Tout particulièrement, la neurostimulation électrique est apparue récemment comme une solution thérapeutique prometteuse pour d'autres pathologies, notamment dans la maladie de Parkinson. Suivant cette optique, à la destruction mécanique d'une région cérébrale prédéfinie, s'est substitué le principe d'un traitement conservateur par stimulations électriques pour renforcer ou inhiber une activité neuronale. La possibilité d'une anticipation des crises que permet le procédé de l'invention est à cet égard décisive, puisqu'elle donne une réponse à la question du « quand stimuler ? ». En effet, ces stimulations peuvent être appliquées lorsqu'un état préictal est détecté et visent à déstabiliser les processus épileptogènes avant que ceux-ci ne deviennent irréversibles au moment de la crise. C'est l'approche que l'on désire avoir sur un plus long terme chez les patients investigués par électrodes intracérébrales.

D'autre part, la technique d'analyse des synchronisations de l'électroencéphalographie peut permettre les développements d'interventions « cognitives ». En effet, certains patients décrivent la faculté qu'ils ont d'interrompre leur crise débutante par des activités cognitives spécifiques ou par des activités motrices. Ces phénomènes reposent vraisemblablement sur une déstabilisation du processus épileptique par l'apparition de nouvelles activités électriques au sein du cortex cérébral. La modulation d'une activité épileptique par des synchronisations cognitives a été également démontrée par les inventeurs.

D'autres interventions peuvent également être appliquées, par exemple l'intervention pharmacologique, consistant en l'administration d'un médicament antiépileptique d'action rapide (comme les benzodiazépines).

Ces possibilités d'alerte et d'interventions, offertes par l'anticipation des crises, impliquent nécessairement l'anticipation en « temps réel », c'est-à-dire que les résultats des calculs mathématiques soient comme dans le procédé de l'invention obtenus instantanément, et non de façon différée.

La capacité d'anticipation des crises permet également d'améliorer la réalisation d'examens effectués lors du bilan préchirurgical des épilepsies partielles pharmacorésistantes. Notamment, la réalisation de la scintigraphie cérébrale précritique (SPECT-ictal) est facilitée par la mise en alerte de l'équipe : l'injection du traceur radioactif au tout début de la crise, voire juste avant, localise mieux le foyer épileptogène. Les temps d'hospitalisation peuvent être alors considérablement réduits et le temps d'occupation des systèmes d'imagerie optimisé. La possibilité d'anticiper la survenue des crises d'épilepsie, grâce à l'électroencéphalographie de profondeur et de surface ouvre des perspectives très vastes en application sociale et clinique.

Procédure mathématique utilisée pour le calcul de synchronie de phase entre deux signaux

La phase instantanée d'un signal peut être calculée soit à l'aide d'un signal analytique, concept introduit par Gabor en 1946 et récemment appliqué sur des données expérimentales, soit par convolution avec une ondelette complexe spécifique (Lachaux et al, Human Brain Mapping, 1999).

Pour un signal arbitraire s(t), le signal analytique $\zeta$ est une fonction complexe dépendant du temps et définie comme suit :

$$\zeta(t) = s(t) + j\tilde{s}(t) = A(t)e^{j\phi(t)} \qquad (1)$$

où la fonction $\tilde{s}(t)$ est la transformée de Hilbert de s(t) :

$$\tilde{s}(t) = \frac{1}{\pi} P.V. \int_{-\infty}^{+\infty} \frac{s(t)}{t - \tau} d\tau \qquad (2)$$

P. V. indique que l'intégrale est calculée au sens de la valeur principale de Cauchy. L'amplitude instantanée A(t) et la phase instantanée $\phi(t)$ du signal s(t) sont uniquement définies par l'équation (1). Comme on peut le voir dans l'équation (2), $\tilde{s}(t)$ est considéré comme le produit de convolution de s(t) et de $1/\pi$. Cela signifie que la transformée de Hilbert est équivalente à un filtre dont la réponse en amplitude est unitaire et la réponse en phase décalée de $\pi/2$ pour toutes les fréquences. Si cette transformée peut s'appliquer en théorie à des signaux à large bande de fréquence, la notion de phase dans ce cas n'est pas très claire. En pratique seulement des signaux à bande étroite obtenus par filtrage sont utilisés. En conséquence, un filtrage est toujours effectué dans une bande de fréquence spécifique. Plusieurs bandes de fréquence peuvent être retenues. La même bande de fréquence est utilisée pour deux signaux en cas d'une synchronie 1:1. Des bandes de fréquence différentes sont employées pour l'étude des synchronies n:m. Le niveau statistique de la synchronisation PLS entre deux signaux est évalué à l'aide d'un des deux indices suivants :

- la variance circulaire de la différence de phase ($\Delta\phi$) entre les signaux ; ou
- l'entropie normalisée de Shannon de cette différence de phase ($\Delta\phi$).

La variance circulaire est telle que :

$$VC = \left| \sum_{k=1}^{M} e^{(i\Delta\phi k)} \right|$$

L'entropie normalisée de Shannon est telle que :

$$\gamma = (H_{max} - H) / H_{max}$$

avec l'entropie H définie par :

$$H = -\sum_{k=1}^{N} p_k \ln p_k$$

où N est le nombre de classe, $H_{max}=\ln(N)$ l'entropie maximale, et $p_k$ la fréquence relative de la différence de phase dans la $k^{ième}$ classe. Le nombre optimal de classe est $N=\exp[0,626+0, 4\ln(M-1)]$ où M est le nombre d'éléments (différence de phase) à classer. Avec cette normalisation, les valeurs de $\gamma$ sont comprises, pour 95 % des « surrogates » (valeurs de remplacement), entre 0 (distribution uniforme et pas de synchronisation) et 1 (parfaite synchronisation). Ce calcul est fait pour toutes les paires de capteurs enregistrés dans le protocole de montage. Pour 27 électrodes d'un montage standard, le nombre de paires distinctes est de 26x25/2=325 et pour 128 électrodes, il s'élève à 8001.

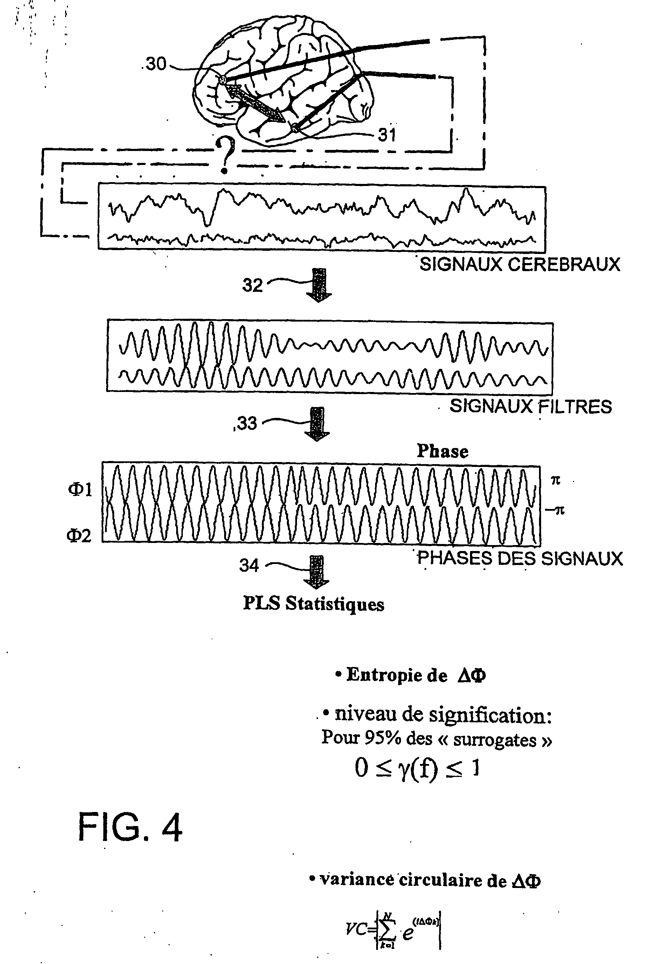

Ainsi, comme illustré sur la figure 4, on a successivement :

- filtrage passe-bande de deux signaux obtenus aux deux électrodes 30 et 31 (f $\pm$ 1 Hz) ;
- transformée de Hilbert de ces signaux ;
- évaluation du niveau statistique de la synchronisation PLS à l'aide de deux indices :

    • entropie de $\Delta\phi$ (différence de phase entre $\phi1$ et $\phi2$) ;
    • variance circulaire de $\Delta\phi$.

La deuxième étape consiste en la mise en place de la base de données des états calibrés du sujet en fonction de l'objectif à atteindre.

La troisième étape est une étape de discrimination à but décisionnel. Etant donné une période d'enregistrement de 10 secondes en pathologogie, mais parfois beaucoup plus courte pour la discrimination d'états cognitifs, dont on connaît la quantification par la méthode de synchronisation, on essaye de l'affecter à une classe, caractérisant un état cérébral parmi plusieurs. C'est un problème de classement, qui suppose que l'on a défini à priori un ensemble de classes. La principale difficulté est la dimension de l'espace des variables. En effet, quantifier la synchronisation entre tous les couples de capteurs et dans 6 bandes de fréquence correspond à un espace de variable de dimension p=1950 (325x6) pour un montage à 27 électrodes. La probabilité a posteriori d'appartenance de la fenêtre temporelle x analysée aux k différents groupes d'états cérébraux a pour expression (théorème de Bayes) :

$$P(G_r \,/\, x) \;=\; P(G_r)\,.P(x\,/\,G_r)\,/\,\sum_{j=1}^{K} P(Gj)\,.P(x\,/\,Gj) \quad \text{avec} \quad r = 1,\ldots,k$$

$P(G_r)$ est la probabilité a priori d'appartenance à une classe et est estimée en pratique par la fréquence des éléments de $G_r$ dans l'échantillon total. Les différentes $P(x/G_r)$ sont estimées par les densités de probabilité. Pour chaque nouvelle période x analysée et à classer dans l'un des k groupes, on recherche les q plus proches voisins de chacun des k groupes en définissant ainsi le rayon moyen $r_k$ de l'hypersphère $HS(r_k,x)$ contenant la moyenne des q voisins de x et le volume $A_k$ de l'hypersphère correspondante dans l'espace $R^p$. Ainsi la densité de probabilité $P(x/G_r)$ peut être estimée par :

$$\hat{P}(x \,/\, G_r) \;=\; \frac{q}{n_r A_r}$$

et x est affecté au groupe $j \in [1, k]$ si: $P(Gj/x) = \max\{P(G_r/x)\;;\;r=1,2,\ldots,k\}$.

Exemple de mise en oeuvre de l'invention

Le dispositif de l'invention a été appliqué aux enregistrements électriques cérébraux intracrâniens de patients relevant d'un traitement chirurgical de leur épilepsie temporale et on a pu ainsi montrer, qu'il est possible d'anticiper les crises de plusieurs minutes et qu'il existe un phénomène déterministe de « route vers la crise », comme illustré sur la figure 4. Le dispositif de l'invention permet une anticipation de la crise dans la bande de fréquence 10-20 Hz de près de 20 minutes avant la crise et est caractérisé par une diminution de synchronisation. Le spectre de puissance, procédure classique en traitement du signal, ne montre pas de claires modifications.

Le procédé de l'invention est spécialement adapté aux situations cliniques et permet en raison d'une faible sensibilité aux artefacts d'enregistrement, d'étendre les résultats à l'électroencéphalographie de surface.

REFERENCES

[1] LE VAN QUYEN M., MARTINERIE J., NAVARRO V., BOON P., D'HAVE M., ADAM C., RENAULT B., VARELA F. ET BAULAC M., « Anticipation of epileptic seizures from standard surface EEG recordings » (2001, The Lancet, 357, pages 183-188).

[2] MARTINERIE J., ADAM C., LE VAN QUYEN M., BAULAC M., CLEMENCEAU S., RENAULT B., VARELA F., « Can epileptic seizure be anticipated by nonlinear analysis » (Nature Medicine 1998, vol. 4, 10, pages 1173-1176).

[3] VARELA F. , LACHAUX J. P., RODRIGUEZ E. et MARTINERIE J., « The Brainweb : Phase synchronization and Large-scale integration » (Nature Reviews Neuroscience 2001, 2, pages 229-239).

[4] LE VAN QUYEN M., FOUCHER J., LACHAUX J.-P., RODRIGUEZ E., LUTZ A., MARTINERIE J., ET VARELA F., « Comparison of Hilbert transform and wavelet methods for the analysis of neuronal synchrony» (2001, Journal of Neuroscience Methods, 111, pages 83 98).

**Revendications**

1. Procédé d'analyse des synchronisations de l'électroencéphalographie d'un individu en utilisant un ensemble de capteurs à partir de l'analyse électromagnétique cérébrale de celui-ci, comprenant les étapes suivantes :

   - une étape de constitution d'une base de données (12) comprenant :

      • une phase (10) d'acquisition et de numérisation de signaux électrophysiologiques issus de ces capteurs ;
      • une phase (11) de calcul d'une corrélation entre des variations temporelles de phase instantanée entre toutes les paires de capteurs enregistrés dans un protocole de montage, dans des bandes de fréquences comprises entre 0 et 2000 Hz, pour constituer cette base de données (12) de classes caractérisant chacune un état de référence ;

   - une étape (13) de validation statistique d'une période analysée en temps réel basée sur une méthode de discrimination non paramétrique multidimensionnelle, qui permet d'affecter cette période à une classe de la base de données ; et
   - une étape (14) de détection d'une période cognitive ou d'une période pathologique spécifique.

2. Procédé selon la revendication 1, qui comprend une analyse associée à au moins l'un des types de signaux électrophysiologiques suivants : électrocardiogrammes, électroocculogrammes, électrodermogrammes, signaux de respiration.

3. Procédé selon la revendication 1, qui, lors de l'étape de validation statistique, utilise une méthode dite « statistiques de verrouillage de phase », ou PLS, qui estime la différence de phase entre les oscillations des signaux de deux électrodes.

4. Procédé selon la revendication 3, dans lequel le niveau statistique de corrélation PLS entre des variations de phase entre deux signaux est évalué à l'aide de la variance circulaire de la différence de phase entre les signaux.

5. Procédé selon la revendication 3, dans lequel le niveau statistique de corrélation PLS entre des variations de phase entre deux signaux est évalué à l'aide de l'entropie normalisée de Shannon de la différence de phase entre les signaux.

6. Application du procédé selon l'une quelconque des revendications 1 à 5 à un suivi cognitif en temps réel.

7. Dispositif de suivi médical ou cognitif en temps réel à partir de l'analyse électromagnétique cérébrale d'un individu comprenant:

   - des moyens (10) d'acquisition et de numérisation de signaux électrophysiologiques issus de capteurs ;
   - des moyens (11) de calcul d'une corrélation entre des variations temporelles de phase instantanée entre toutes les paires de capteurs enregistrés dans un protocole de montage, dans des bandes de fréquences comprises entre 0 et 2000 Hz, pour constituer une base de données (12) de classes caractérisant chacune un état de référence ;
   - des moyens (13) de validation statistique, par une méthode de discrimination non paramétrique multidimensionnelle, d'une période analysée en temps réel, qui permet d'affecter cette période à une classe de la base de données ;
   - des moyens (14) de détection d'une période cognitive ou d'une période pathologique spécifique ; et

- des moyens (15) d'émission éventuelle d'un signal d'alerte.

8.  Dispositif selon la revendication 7, qui comprend des moyens d'analyse associée à au moins l'un des types de signaux électrophysiologiques suivants : électrocardiogrammes, électroocculogrammes, électrodermogrammes, signaux de respiration.

9.  Dispositif selon la revendication 7, dans lequel les moyens de validation statistique utilisent une méthode dite « statistiques de verrouillage de phase », ou PLS, qui estime la différence de phase entre les oscillations des signaux de deux électrodes.

10. Dispositif selon la revendication 9, dans lequel le niveau statistique de corrélation PLS entre des variations de phase entre deux signaux est évalué à l'aide de la variance circulaire de la différence de phase entre les signaux.

11. Dispositif selon la revendication 9, dans lequel le niveau statistique de corrélation PLS entre des variations de phase entre deux signaux est évalué à l'aide de l'entropie normalisée de Shannon de la différence de phase entre les signaux.

12. Dispositif selon la revendication 7, qui comprend :

    - des circuits (20,21, 22) d'acquisition des signaux de l'activité électrique du cerveau ;
    - un processeur (23) permettant l'acquisition et le traitement de ces signaux ;
    - un circuit d'alerte pour l'individu ou son environnement.

13. Dispositif selon la revendication 7, qui est un dispositif transportable par ledit individu.

14. Dispositif selon la revendication 7, qui est miniaturisé pour pouvoir être implanté en sous-cutané.


**Claims**

1.  A method of analyzing synchronizations of electroencephalography of an individual using a set of sensors starting from cerebral electromagnetic analysis of the individual, comprising the following steps:

    - a step of creating a database (12) comprising:

        • a step (10) of acquiring and digitizing electrophysiological signals output from the sensors,
        • a step (11) of calculating a correlation of temporal variations of instantaneous phase between all pairs of sensors recorded in an assembly protocol, in frequency bands between 0 and 2000 Hz, to build up the database (12) of classes, each characterizing a reference state;

    - a step (13) of statistical validating a period analyzed in real time, based on a non-parametric multidimensional discrimination method which allows assigning said period to a class of the database; and
    - a step (14) of detecting a specific cognitive or pathologic period.

2.  A method according to claim 1, comprising an analysis associated with at least one type of electrophysiological signals among the following: electrocardiograms, electrooculograms, electrodermograms, breathing signals.

3.  A method according to claim 1, wherein a PLS - Phase Locking Statistics - method is used during the statistical validation step, which estimates a phase difference between oscillations of signals from two electrodes.

4.  A method according to claim 3, wherein a statistical level of PLS correlation between the phase variations of two signals is evaluated using circular variance of the phase difference between the signals.

5.  A method according to claim 3, wherein a statistical level of PLS correlation between two signals is evaluated using normalized Shannon entropy of the phase difference between the signals.

6.  Application of the method according to any of claims 1 to 5 to real time cognitive monitoring.

**7.** A real time medical or cognitive monitoring device starting from cerebral electromagnetic analysis of an individual, comprising:

- means (10) for acquiring and digitizing electrophysiological signals output from sensors;
- means (11) for calculating correlation of temporal variations of instantaneous phase between all pairs of sensors recorded in an assembly process, in frequency bands between 0 and 2000 Hz, to build up a database (12) of classes each characterizing a reference state;
- means (13) for statistically validating, by a non-parametric multidimensional discrimination method, a period analyzed in real time to assign the period to a class in the database;
- means (14) for detecting a cognitive period or a specific pathological period; and
- means (15) for sending an alert signal if applicable.

**8.** A device according to claim 7, comprising means for performing an analysis associated with at least one type of electrophysiological signals among the following: electrocardiograms, electrooculograms, electrodermograms, breathing signals.

**9.** A device according to claim 7, wherein a PLS - Phase Locking Statistics - method is used during the statistical validation step, which estimates a phase difference between oscillations of signals from two electrodes.

**10.** A device according to claim 9, wherein a statistical level of a PLS correlation between two signals is evaluated using circular variance of the phase difference between the signals.

**11.** A device according to claim 9, wherein a statistical level of PLS correlation between two signals is evaluated using normalized Shannon entropy of the phase difference between the signals.

**12.** A device according to claim 7, further comprising:

- circuits (20, 21, 22) for acquisition of signals representing electrical activity of the brain;
- a processor (23) configured for acquisition and processing of the signals;
- and an alert circuit for the patient or for his/her environment.

**13.** A device according to claim 7, which is a device that the individual can carry himself or herself.

**14.** A device according to claim 7, miniaturized to be implanted subcutaneously.


**Patentansprüche**

**1.** Verfahren zur Analyse von Synchronisationen der Elektroenzephalografie eines Individuums unter Benutzung einer Gesamtheit von Sensoren ausgehend von der zerebralen elektromagnetischen Analyse des Individuums, umfassend die folgenden Schritte:

- ein Schritt der Bildung einer Datenbasis (12) umfassend:

• eine Phase (10) der Erfassung und der Digitalisierung der von den Sensoren ausgehenden elektrophysiologischen Signale;
• eine Phase (11) der Berechnung einer Korrelation zwischen zeitlichen Variationen der momentanen Phase zwischen allen Paaren von Sensoren, welche in einem Anbringungsprotokoll registriert sind, in Frequenzbändern, welche zwischen 0 und 2000 Hz enthalten sind, um die Datenbasis (12) von Klassen zu bilden, welche jeweils einen Referenzzustand charakterisieren,

- einen Schritt (13) der statistischen Validierung einer in Realzeit analysierten Periode basierend auf einem nicht parameterischen multidimensionalen Unterscheidungsverfahren, was es erlaubt, diese Periode einer Klasse der Datenbasis zuzuordnen, und
- einen Schritt der Detektion einer kognitiven Periode oder spezifischen pathologischen Periode.

**2.** Verfahren gemäß Anspruch 1, welches eine mit zumindest einem der folgenden Typen von elektrophysiologischen Signalen verknüpfte Analyse umfasst: Elektrokardiogramme, Elektrookulogramme, Elektrodermogramme, Atmungssignale.

3. Verfahren gemäß Anspruch 1, welches während dem Schritt der statistischen Validierung eine Methode, welche "Statistiken der Verriegelung der Phase" oder PLS genannt wird, welche die Phasendifferenz zwischen den Oszillationen der Signale der zwei Elektroden schätzt, benutzt.

4. Verfahren gemäß Anspruch 3, bei welchem das statistische Niveau der PLS-Korrelation zwischen den Variationen der Phase zwischen zwei Signalen mithilfe der zirkularen Varianz der Phasendifferenz zwischen den Signalen ausgewertet wird.

5. Verfahren gemäß Anspruch 3, bei welchem das statistische Niveau der PLS-Korrelation zwischen den Variationen der Phase zwischen zwei Signalen mithilfe der normalisierten Shannon-Entropie der Phasendifferenz zwischen den Signalen ausgewertet wird.

6. Anwendung des Verfahrens gemäß einem der Ansprüche 1-5 auf eine kognitive Betreuung in Realzeit.

7. Vorrichtung zur medizinischen oder kognitiven Betreuung in Realzeit ausgehend von der elektromagnetischen zerebralen Analyse eines Individuums, umfassend:

- Mittel (10) zur Erfassung und zur Digitalisierung von von Sensoren ausgehenden elektrophysiologischen Signalen,
- Mittel (11) zur Berechnung einer Korrelation zwischen zeitlichen Variationen der momentanen Phase zwischen allen Paaren von Sensoren, welche in einem Anbringungsprotokoll registriert sind, in zwischen 0 und 2000 Hz enthaltenen Frequenzbändern, um eine Datenbasis (12) von Klassen zu bilden, welche jeweils einen Referenzzustand charakterisieren,
- Mittel (13) zur statistischen Validierung einer in Realzeit analysierten Periode durch ein nicht parameterisches multidimensionales Unterscheidungsverfahren, was es erlaubt, diese Periode einer Klasse der Datenbasis zuzuordnen,
- Mittel (14) zur Detektion einer kognitiven Periode oder einer spezifischen pathologischen Periode, und
- Mittel (15) zur möglichen Aussendung eines Alarmsignals.

8. Vorrichtung gemäß Anspruch 7, welche Analysemittel umfasst, welche mindestens einem der folgenden elektrophysiologischen Signaltypen zugeordnet sind: Elektrokardiogramme, Elektrookulogramme, Elektrodermogramme, Atmungssignale.

9. Vorrichtung gemäß Anspruch 7, in welcher die Mittel zur statistischen Validierung ein "Statistiken der Verriegelung der Phase" oder PLS genanntes Verfahren benutzen, welches die Phasendifferenz zwischen den Oszillationen der Signale der zwei Elektroden schätzt.

10. Vorrichtung gemäß Anspruch 9, bei welcher das statistische Niveau der PLS-Korrelation zwischen den Variationen der Phase zwischen zwei Signalen mithilfe der zirkularen Varianz der Phasendifferenz zwischen den Signalen ausgewertet wird.

11. Vorrichtung nach Anspruch 9, bei welcher das statistische Niveau der PLS-Korrelation zwischen den Variationen der Phase zwischen zwei Signalen mithilfe der normalisierten Shannon-Entropie der Phasendifferenz zwischen den Signalen ausgewertet wird.

12. Vorrichtung gemäß Anspruch 7, welche umfasst:

- Schaltungen (20, 21, 22) zur Erfassung von Signalen der elektrischen Hirnaktivität,
- einen Prozessor (23), welcher die Erfassung und die Verarbeitung dieser Signale ermöglicht,
- eine Alarmschaltung für das Individuum oder seine Umgebung.

13. Vorrichtung gemäß Anspruch 7, welche eine für das Individuum transportable Vorrichtung ist.

14. Vorrichtung gemäß Anspruch 7, welche miniaturisiert ist, so dass sie subkutan implantiert werden kann.

ACQUISITION ET NUMERISATION
DES SIGNAUX PHYSIOLOGIQUES — 10

CALCUL DE LA SYNCHRONISATION ENTRE
TOUTES LES PAIRES DE SIGNAUX ET DANS
PLUSIEURS BANDES DE FREQUENCE — 11

CONSTITUTION D'UNE BASE DE DONNEES
D'ETATS DE REFERENCE — 12

VALIDATION STATISTIQUE DE LA PERIODE
ANALYSEE EN TEMPS REEL — 13

DETECTION DE PERIODES COGNITIVES
OU DE PERIODES PATHOLOGIQUES SPECIFIQUES — 14

EMISSION EVENTUELLE D'UN
SIGNAL ALARME — 15

FIG. 1

BASE DE DONNEES
k CLASSES

ACQUISITION
DES SIGNAUX

CALCUL DES k PARTITIONS
DE L'ESPACE DES VARIABLES
PAR PROBABILITES
BAYESIENNES

NUMERISATION
DES SIGNAUX

SYNCHRONISATION
ENTRE TOUTES LES
PAIRES DE SIGNAUX

SIGNAUX D'UNE
FENETRE
TEMPORELLE x

FIG. 2

CLASSEMENT DE LA FENETRE x
DANS LA BASE DES k CLASSES

CLASSE A
DETECTER ?

OUI

ALERTE

SORTIE
EXTERNE

NON

FIG. 3

```
        SIGNAUX EEG

      AMPLIFICATEURS          ⟞ 20

   CONVERTISSEUR BLOQUEUR
   ANALOGIQUE / NUMERIQUE     ⟞ 21

      STOCKAGE DONNEES
22 ⟝ DANS MEMOIRE TAMPON

  PROGRAMME → PROCESSEUR      ⟞ 23

                         24 ⟝

   SORTIE EXTERNE        AVERTISSEUR
```

SIGNAUX CEREBRAUX

32

SIGNAUX FILTRES

33

**Phase**

$\Phi 1$    $\pi$

$-\pi$

$\Phi 2$

PHASES DES SIGNAUX

34

**PLS Statistiques**

• **Entropie de** $\Delta\Phi$

• **niveau de signification:**
Pour 95% des « surrogates »

$$0 \leq \gamma(f) \leq 1$$

# FIG. 4

• **variance circulaire de** $\Delta\Phi$

$$VC = \left| \sum_{k=1}^{N} e^{(i\Delta\Phi_k)} \right|$$

EP 1 551 288 B1

FIG. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4846190 A

**Littérature non-brevet citée dans la description**

- **LE VAN QUYEN M. ; MARTINERIE J. ; NAVARRO V. ; BOON P. ; D'HAVE M. ; ADAM C. ; RENAULT B. ; VARELA F. ; BAULAC M.** Anticipation of epileptic seizures from standard surface EEG recordings. *The Lancet,* 2001, vol. 357, 183-188
- **MARTINERIE J. ; ADAM C. ; LE VAN QUYEN M. ; BAULAC M. ; CLEMENCEAU S. ; RENAULT B. ; VARELA F.** Can epileptic seizure be anticipated by nonlinear analysis. *Nature Medicine,* 1998, vol. 4 (10), 1173-1176
- **VARELA F. ; LACHAUX J. P. ; RODRIGUEZ E. ; MARTINERIE J.** The Brainweb : Phase synchronization and Large-scale integration. *Nature Reviews Neuroscience,* 2001, vol. 2, 229-239
- **LE VAN QUYEN M. ; FOUCHER J. ; LACHAUX J.-P. ; RODRIGUEZ E. ; LUTZ A. ; MARTINERIE J. ; ET VARELA F.** Comparison of Hilbert transform and wavelet methods for the analysis of neuronal synchrony. *Journal of Neuroscience Methods,* 2001, vol. 111, 83-98